(19) Europäisches Patentamt
European Patent Office
Office européen des brevets

(11) **EP 4 636 095 A1**

(12) **EUROPEAN PATENT APPLICATION**
published in accordance with Art. 153(4) EPC

(43) Date of publication:
**22.10.2025 Bulletin 2025/43**

(21) Application number: **22968291.9**

(22) Date of filing: **16.12.2022**

(51) International Patent Classification (IPC):
***C12Q 1/68*** *(2018.01)*

(52) Cooperative Patent Classification (CPC):
**C12Q 1/68**

(86) International application number:
**PCT/CN2022/139798**

(87) International publication number:
**WO 2024/124587 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **BGI Genomics Co., Limited
Guangdong 518083 (CN)**
• **Shijiazhuang BGI Clinical Laboratory Co., Ltd.
Shijiazhuang, Hebei 050035 (CN)**

(72) Inventors:
• **ZHOU, Si
Shenzhen, Guangdong 518083 (CN)**

• **ZHAO, Lijian
Shenzhen, Guangdong 518083 (CN)**
• **ZHANG, Jianguo
Shenzhen, Guangdong 518083 (CN)**
• **YANG, Wenzhi
Shenzhen, Guangdong 518083 (CN)**
• **PENG, Huanhuan
Shenzhen, Guangdong 518083 (CN)**

(74) Representative: **Inspicos P/S
Agern Allé 24
2970 Hørsholm (DK)**

Remarks:
The complete document including Reference
Table(s) and the Sequence Listing(s) can be
downloaded from the EPO website

(54) **PREECLAMPSIA BIOMARKER AND USE THEREOF**

(57)    Provided are a biomarker for predicting a pre-eclampsia risk, use thereof, and a method for detecting whether a subject has a preeclampsia risk. The method predicts the preeclampsia risk in all pregnant women in the early and middle stages of pregnancy, without distinguishing the population with a high preeclampsia risk. The method can predict the risk before symptoms occur. A variety of free RNAs in the blood are associated with preeclampsia, and by establishing a preeclampsia prediction model with samples from at least 300 cases using the biomarker, the present disclosure can improve the specificity of the preeclampsia prediction model during the pregnancy to 85% or higher.

Figure 1

## Description

## Technical Field

The present disclosure relates to the field of biomedical technology, and specifically, the present disclosure relates to a preeclampsia biomarker and use thereof.

## Background

Preeclampsia (PE) is a pregnancy disease associated with new-onset hypertension during pregnancy, affecting 3% to 5% of pregnancies. In the latest guidelines, preeclampsia is defined that a pregnant women has a systolic blood pressure ≥140 mmHg and/or a diastolic blood pressure ≥90 mmHg after 20 weeks of pregnancy, accompanied by any one of the following: urine protein quantification ≥0.3 g/24 h, or urine protein/creatinine ratio ≥0.3, or random urine protein ≥(+) (examination method when protein quantification is unconditional); no proteinuria but accompanied by any one of the following organs or systems involved: important organs such as the heart, lungs, liver, and kidneys, or abnormal changes in the blood system, digestive system, and nervous system, placenta-fetus involvement, etc. The FIGO guidelines divide preeclampsia into four types according to the time of preeclampsia diagnosis and the time of delivery: earlyonset preeclampsia, late-onset preeclampsia, premature delivery, and full-term preeclampsia.

To date, the exact pathogenesis of preeclampsia remains unclear, and there is no effective treatment. Termination of pregnancy and placental delivery are the only effective treatment options for preeclampsia. Among the measures to prevent preeclampsia, taking aspirin for pregnant women at high risk of preeclampsia is a recognized method (Poon LC, Shennan A, Hyett JA, et al. The International Federation of Gynecology and Obstetrics (FIGO) initiative on preeclampsia: A pragmatic guide for first-trimester screening and prevention. Int J Gynaecol Obstet. 2019;145 Suppl 1:1-33. Obstetricians ACo and Gynecologists. Gestational hypertension and preeclampsia: ACOG Practice Bulletin, number 222. Obstet Gynecol. 2020;135:e237-e60). Studies have shown that taking aspirin at ≤16 weeks of pregnancy can significantly reduce the risk of preeclampsia (Villa PM, Kajantie E, Räikkönen K, et al. Aspirin in the prevention of preeclampsia in high-risk women: a randomised placebo-controlled PREDO Trial and a meta-analysis of randomised trials. BJOG: An International Journal of Obstetrics & Gynaecology. 2013;120:64-74). How to make early predictions for pregnant women at high risk of preeclampsia before the onset of preeclampsia is an urgent problem to be solved.

Some risk factors for preeclampsia are known, such as advanced age; family history and history of preeclampsia; pregnancy interval; obesity, etc., for screening of high-risk populations for preeclampsia. However, due to the heterogeneity and complexity of preeclampsia, the absence of known risk factors does not mean that preeclampsia will not occur. It is not accurate to predict the high-risk population for preeclampsia through maternal risk factors. Placental cell dysfunction can lead to serious pregnancy complications, but invasive placental tissue sampling can cause certain insecurity to pregnant women and fetuses. By analyzing free circulating RNA in maternal blood, the functional abnormalities of extravillous trophoblastic cells in the placenta of preeclampsia can be non-invasively found, which helps to predict preeclampsia in pregnant women before the onset of preeclampsia symptoms.

In 2019, the published patent "Circulating RNA markers specific to preeclampsia" (Publication No.: CN110785499A) of ILLUMINA INC proposes a method for detecting preeclampsia and/or determining an increased risk of preeclampsia in pregnant women, the method comprising identifying multiple circulating RNA (C-RNA) molecules in a biological sample obtained from the pregnant woman. The patent is based on the analysis of the confirmed population, and these C-RNAs are used to build a model for classification, not prediction.

The patent "Mean and method for excluding the occurrence of preeclampsia within a certain period of time by using the ratio of sFlt-1/PlGF or endoglin/PlGF" (patent CN104412107B) discloses a method for diagnosing whether a pregnant subject is not at risk of preeclampsia within a short time window. The method is based on the ratio of sFlt-1/PlGF or endoglin/PlGF to predict preeclampsia, which can only predict whether there is a risk of preeclampsia in a short period of time, and has certain limitations.

Therefore, so far, there is no widely recognized biomarker that can be used in clinical practice to predict various types of preeclampsia. Therefore, it is urgent to develop a non-invasive biomarker that can be widely used in clinical practice and can be applied to various stages of pregnancy with high accuracy to predict preeclampsia early before the onset of disease symptoms.

## Contents of the present invention

The present disclosure aims to solve one of the technical problems in the related art at least to a certain extent. To this end, one purpose of the present disclosure is to provide a biomarker combination for predicting the risk of preeclampsia and a method for detecting whether a subject has the risk of preeclampsia. The method for detecting whether a subject has the risk of preeclampsia provided by the present disclosure can predict the risk of preeclampsia for all pregnant women in the early and middle stages of pregnancy, regardless of whether they are high-risk groups for preeclampsia, and can predict the risk before the onset of symptoms; and can discover the association between different types of cell-free RNA in the blood and preeclampsia. By constructing a preeclampsia prediction model in at least 300 samples, the specificity of the prediction model for preeclampsia during pregnancy is improved to more than 85%.

In one aspect of the present disclosure, the present disclosure provides a biomarker combination for predicting the risk of preeclampsia. According to one embodiment of the present disclosure, the biomarker combination comprises the following genes:

ALB, FGA, MIR27A, IGFBP5, MIR376C, MIR215, SERPINA1 and MIR106A.

According to one embodiment of the present disclosure, the biomarker combination is a combination of RNA molecular markers;

preferably, the biomarker combination is a combination of cfRNA (cell-free RNA) molecular marker.

According to one embodiment of the present disclosure, the biomarker combination comprises:

an mRNA transcript of ALB, an mRNA transcript of FGA, an miRNA transcript of MIR27A, an mRNA transcript of IGFBP5, an miRNA transcript of MIR376C, an miRNA transcript of MIR215, an mRNA transcript of SERPINA1 and an miRNA transcript of MIR106A.

According to one embodiment of the present disclosure, the biomarker combination further comprises at least one of the following genes:

MIR190A, S100A9, APOA1, MALAT1, CGA, LEP, MIR130A, MIR144, MIR19B1 and MIR33A.

According to one embodiment of the present disclosure, the biomarker combination further comprises at least one of the following:

an miRNA transcript of MIR190A, an mRNA transcript of S100A9, an mRNA transcript of APOA1, an lcnRNA transcript of MALAT1, an mRNA transcript of CGA, an miRNA transcript of MIR19B1, an miRNA transcript of MIR144, an miRNA transcript of MIR33A, an miRNA transcript of MIR130A and an mRNA of LEP.

According to one embodiment of the present disclosure, the biomarker combination comprises:

MIR190A, ALB, FGA, MIR27A, IGFBP5, MIR376C, S100A9, APOA1, MALAT1, MIR215, CGA, SERPINA1 and MIR106A.

According to a preferred embodiment of the present disclosure, the biomarker combination comprises: an miRNA transcript of MIR190A, an mRNA transcript of ALB, an mRNA transcript of FGA, an miRNA transcript of MIR27A, an mRNA transcript of IGFBP5, an miRNA transcript of MIR376C, an mRNA transcript of S100A9, an mRNA transcript of APOA1, an lcnRNA transcript of MALAT1, an miRNA transcript of MIR215, an mRNA transcript of CGA, an mRNA transcript of SERPINA1 and an miRNA transcript of MIR106A.

According to an embodiment of the present disclosure, the biomarker combination comprises:

MIR19B1, ALB, FGA, MIR27A, IGFBP5, MIR376C, MIR144, MIR33A, MIR130A, MIR215, LEP, SERPINA1 and MIR106A.

According to a preferred embodiment of the present disclosure, the biomarker combination comprises: an miRNA transcript of MIR19B1, an mRNA transcript of ALB, an mRNA transcript of FGA, an miRNA transcript of MIR27A, an mRNA transcript of IGFBP5, an miRNA transcript of MIR376C, an miRNA transcript of MIR144, an miRNA transcript of MIR33A, an miRNA transcript of MIR130A, an miRNA transcript of MIR215, an mRNA of LEP, an mRNA transcript of SERPINA1 and an miRNA transcript of MIR106A.

According to an embodiment of the present disclosure, the biomarker combination further comprises: a clinical phenotype;

preferably, the clinical phenotype comprises at least one of the followings:

whether in vitro fertilization is performed, mean arterial pressure, BMI, maternal age, parity, and miscarriage history. BMI=weight ÷ height$^2$.

The second aspect of the present disclosure provides a reagent for detecting the biomarker combination as described in the first aspect, wherein the reagent comprises: a biomolecule capable of specifically hybridizing with any one of the biomarkers in the biomarker combination or their expression products and/or a detection reagent that uses the biomarker combination as a detection target.

According to one embodiment of the present disclosure, the biomolecule comprises at least one of a primer, a probe and an antibody.

The third aspect of the present disclosure provides a detection product, comprising the reagent as described in the second aspect.

According to one embodiment of the present disclosure, the detection product comprises a kit and/or a chip.

The fourth aspect of the present disclosure provides a method for detecting preeclampsia or predicting the risk of preeclampsia. According to one embodiment of the present disclosure, the method comprises:

(1) obtaining a biological sample to be detected;

(2) determining the detection data of the biomarker combination as described in the first aspect in the biological sample obtained in step (1);

(3) identifying whether there is a preeclampsia or a risk of preeclampsia based on the detection data described in step (2),

wherein the detection data of the biomarker combination comprises the expression level of a gene and optionally a clinical phenotype result.

When the biomarker combination is a gene, the detection data is the expression level of the gene. When the biomarker combination also comprises a clinical phenotype, the detection data also comprises a clinical phenotype result.

According to one embodiment of the present disclosure, the biological sample comprises one or more of plasma, whole blood, amniotic fluid, serum and urine.

According to one embodiment of the present disclosure, the biological sample is collected before the 33$^{rd}$ gestational week (including the 33$^{rd}$ gestational week) of a pregnant woman, preferably collected from the 12$^{th}$ to 33$^{rd}$ gestational weeks.

According to one embodiment of the present disclosure, when the biological sample is a blood sample, the biological sample is derived from a peripheral blood sample of the pregnant woman;

according to one embodiment of the present disclosure, the identification of whether there is a preeclampsia or a risk of preeclampsia is achieved through a prediction model;

according to one embodiment of the present disclosure, the prediction model uses the detection data of the biomarker combination of preeclampsia samples and non-preeclampsia samples as input, and uses the risk score of preeclampsia risk as output, and is obtained by machine learning model training; preferably, the threshold of the risk score is 0.5; preferably, the risk score greater than the threshold indicates that the pregnant woman has preeclampsia or is at risk of preeclampsia.

According to one embodiment of the present disclosure, the machine learning model comprises: one or more of an average neural network model, a gradient boosting machine, a logistic regression model, a neural network model, and a support vector machine, and more preferably an average neural network model or a support vector machine.

According to one embodiment of the present disclosure, the expression level of genes is RNA expression level of genes.

According to one embodiment of the present disclosure, the RNA expression level of the biomarker is determined by quantitative analysis of cfRNA in the biological sample; preferably, the quantitative analysis method comprises a high-throughput sequencing method or an RT-PCR method.

The fifth aspect of the present disclosure provides a device for detecting preeclampsia or predicting the risk of preeclampsia, which comprises:

an acquisition module for acquiring a biological sample to be detected;

a detection module for determining the detection data of the biomarker combination as described in the first aspect in the biological sample;

an identification module for identifying whether there is a preeclampsia or a risk of preeclampsia based on the detection data of the biomarker combination.

When the biomarker combination is a gene, the detection data is the expression level of the gene, and when the biomarker combination also comprises a clinical phenotype, the detection data also comprises a clinical phenotype result.

According to one embodiment of the present disclosure, the biological sample comprises one or more of plasma, whole blood, amniotic fluid, serum and urine.

According to one embodiment of the present disclosure, the biological sample is collected before the 33$^{rd}$ gestational week (including the 33$^{rd}$ gestational week) of a pregnant woman, preferably collected between the 12$^{th}$ and 33$^{rd}$ gestational weeks.

According to one embodiment of the present disclosure, when the biological sample is a blood sample, the biological sample is derived from a peripheral blood sample of a pregnant woman;

according to one embodiment of the present disclosure, the identification of whether there is a preeclampsia or a risk of preeclampsia is achieved through a prediction model;

according to one embodiment of the present disclosure, the prediction model uses the detection data of the biomarker combination of preeclampsia samples and non-preeclampsia samples as input, and uses the risk score of having preeclampsia risk as output, and is obtained by machine learning model training; preferably, the threshold of the risk score is 0.5; preferably, the risk score greater than the threshold indicates that the pregnant woman has preeclampsia or is at risk of preeclampsia.

According to one embodiment of the present disclosure, the machine learning model comprises: one or more of an average neural network model, a gradient boosting machine, a logistic regression model, a neural network model, and a support vector machine, more preferably an average neural network model or a support vector machine.

According to one embodiment of the present disclosure, the expression level of genes is the RNA expression level of genes.

According to one embodiment of the present disclosure, the RNA expression level of the biomarker is determined by quantitative analysis of cfRNA in the biological sample; preferably, the quantitative analysis method comprises a high-throughput sequencing method or an RT-PCR method.

In a sixth aspect, the present disclosure provides a device for detecting preeclampsia or predicting the risk of preeclampsia, wherein the device comprises a memory for storing a program; and a processor for executing the program stored in the memory to implement the method as described in the fourth aspect.

In a seventh aspect, the present disclosure provides a computer-readable storage medium, on which a program is stored, and the program can be executed by a processor to implement the method as described in the fourth aspect.

The eighth aspect of the present disclosure provides use of a biomarker as a target for screening a drug for treating or preventing preeclampsia, wherein the biomarker comprises the biomarker combination as described in the first aspect.

The ninth aspect of the present disclosure provides use of a biomarker in diagnosing or predicting whether a subject has the risk of preeclampsia, wherein the biomarker comprises the biomarker combination as described in the first aspect.

The biomarker combination and method for detecting the risk of preeclampsia or predicting the risk of preeclampsia provided by the present disclosure can predict the risk of preeclampsia for all pregnant women in the early and middle stages of pregnancy, regardless of whether they are high-risk groups for preeclampsia, and can predict the risk before the onset of symptoms;

the association between different types of cell-free RNA in the blood and preeclampsia is discovered, and a preeclampsia prediction model is constructed in at least 300 samples, and the specificity of the prediction model for preeclampsia during pregnancy is increased to more than 85%;

based on the constructed preeclampsia prediction model, at least 150 completely independent samples are used to verify the effect of the prediction model, and the area under the receiver operating characteristic curve (AUC) of the validation set is ≥0.75, and the specificity is >80%.

Additional aspects and advantages of the present disclosure will be partially given in the following description, partially become apparent from the following description, or be understood through the practice of the present disclosure.

**Brief Description of the Drawings**

The above and/or additional aspects and advantages of the present disclosure will become apparent and easy to understand from the description of the examples in conjunction with the following drawings, wherein:

Figure 1 shows the effect of the molecular markers on the prediction model of preeclampsia risk in Example 1 of the present disclosure, in which SVM model verification was performed in 380 samples;

Figure 2 shows the effect evaluation of the prediction model of preeclampsia risk in Example 1 of the present disclosure, i.e., the independent verification results in the first group of 262 cases;

Figure 3 shows the effect evaluation of the prediction model of preeclampsia risk in Example 1 of the present disclosure, i.e., the independent verification results in the second group of 288 cases;

Figure 4 shows the effect of the molecular markers on the prediction model of preeclampsia risk in Example 2 of the present disclosure, in which the AvNN model verification was performed in 430 samples;

Figure 5 shows the effect evaluation of the prediction model for preeclampsia risk in Example 2 of the present disclosure, i.e, the independent verification results in the first group of 288 cases;

Figure 6 shows the effect evaluation of the prediction model for preeclampsia risk in Example 2 of the present disclosure, i.e., the independent verification results in the second group of 197 cases;

Figure 7 shows the effect of the molecular markers on the prediction model of preeclampsia risk in Example 3 of the present disclosure, in which the AvNN model verification was performed in 430 samples;

Figure 8 shows the effect evaluation of the prediction model for preeclampsia risk in Example 3 of the present disclosure, i.e., the independently verification results in the second group of 288 cases;

Figure 9 shows the effect of the molecular markers on the prediction model of preeclampsia risk in Comparative Example 1 of the present disclosure, in which the GBM model verification was performed.

## Specific Models for Carrying Out the present invention

The examples of the present disclosure are described in detail below. The examples described below are exemplary and are only used to explain the present disclosure, and cannot be understood as limiting the present disclosure.

It should be noted that the terms "first" and "second" are only used for descriptive purposes, and cannot be understood as indicating or implying relative importance or implicitly indicating the number of technical features indicated. Thus, the features defined as "first" and "second" may explicitly or implicitly include one or more of the features. Further, in the description of the present disclosure, unless otherwise specified, "multiple" means two or more.

The endpoints and any values of the ranges disclosed in this document are not limited to the precise ranges or values, and these ranges or values should be understood to include values close to these ranges or values. For numerical ranges, the endpoint values of each range, the endpoint values of each range and the individual point values, and the individual point values can be combined with each other to obtain one or more new numerical ranges, which should be regarded as specifically disclosed herein.

In order to make it easier to understand the present disclosure, certain technical and scientific terms are specifically defined below. Unless it is obvious that they are otherwise clearly defined elsewhere herein, all other technical and scientific terms used herein have the meanings generally understood by those of ordinary skill in the art to which the present disclosure belongs.

As used herein, the term "comprising" or "including" is an open expression, that is, including the content specified in the present disclosure, but not excluding other aspects.

As used herein, the term "optionally", "optional" or "option" generally means that the event or condition described subsequently may but may not occur, and the description comprises situations in which the event or condition occurs, as well as situations in which the event or condition does not occur.

In the present disclosure, plasma cell-free RNA is used to find risk prediction molecular markers before the occurrence of preeclampsia, non-invasive method is used to predict the risk of preeclampsia in advance, and a preeclampsia risk prediction model is constructed. The abovescreened biomarker combination can be used to accurately predict the risk of preeclampsia. The inventors used maternal peripheral blood plasma to generate the expression profile of plasma free RNA, compared the differential characteristics of the plasma cell-free RNA expression profiles of pregnant women with preeclampsia and without preeclampsia in the early and mid-pregnancy, screened out preeclampsia risk prediction markers, constructed a preeclampsia risk prediction model using a machine learning algorithm, and finally used completely independent samples to evaluate the effect of the constructed preeclampsia prediction model.

Through the above method, the inventors obtained three biomarker combinations with better effects for risk prediction of preeclampsia: MIR190A, ALB, FGA, MIR27A, IGFBP5, MIR376C, S100A9, APOA1, MALAT1, MIR215, CGA, SERPINA1, MIR106A; MIR19B1, ALB, FGA, MIR27A, IGFBP5, MIR376C, MIR144, MIR33A, MIR130A, MIR215, LEP, SERPINA1, MIR106A; and MIR130A, MIR144, MIR19B1, MIR215, MIR376C, ALB, FGA, MIR27A, LEP, IGFBP5, MIR106A, SERPINA1. The inventors found that these three biomarker combinations all contain 8 genes: ALB, FGA, MIR27A, IGFBP5, MIR376C, MIR215, SERPINA1 and MIR106A (nucleic acid sequences are shown in SEQ ID NOs: 1-8). Therefore, these 8 genes in combination can be used as basic markers for detecting preeclampsia or predicting the risk of preeclampsia. When these 8 genes in combination are supplemented with at least one of the genes MIR190A, S100A9, APOA1, MALAT1, CGA, LEP, MIR130A, MIR144, MIR19B1 and MIR33A (nucleic acid sequences are shown in SEQ ID NOs: 9-18), the gene combination obtained thereby as a biomarker combination could achieve higher accuracy for detecting preeclampsia or predicting the risk of preeclampsia. The 8 genes in combination as basic markers can synergize with an additional marker to improve the accuracy of detecting preeclampsia or predicting the risk of preeclampsia.

According to a specific embodiment of the present disclosure, the biomarker combination also comprises: a clinical phenotype. According to a preferred embodiment of the present disclosure, the clinical phenotype comprises at least one of: whether in vitro fertilization is performed, mean arterial pressure, BMI, maternal age, parity, abortion history. The inventors found that the above genotype biomarker combination combined with these clinical phenotypes will be more accurate to detect preeclampsia or predict the risk of preeclampsia.

According to a preferred embodiment of the present disclosure, the biomarker combination comprises:

(1) the following genes:
ALB, FGA, MIR27A, IGFBP5, MIR376C, MIR215, SERPINA1 and MIR106A;

(2) at least one of the following genes:
MIR190A, S100A9, APOA1, MALAT1, CGA, LEP, MIR130A, MIR144, MIR19B1 and MIR33A; and

(3) at least one of whether in vitro fertilization is performed, mean arterial pressure, BMI, maternal age, parity, and history of miscarriage.

According to a specific embodiment of the present disclosure, the biomarker combination comprises:

1) an mRNA transcript of ALB, an mRNA transcript of FGA, an miRNA transcript of MIR27A, an mRNA transcript of IGFBP5, an miRNA transcript of MIR376C, an miRNA transcript of MIR215, an mRNA transcript of SERPINA1 and an miRNA transcript of MIR106A;

2) at least one of the following:
an miRNA transcript of MIR190A, an mRNA transcript of S100A9, an mRNA transcript of APOA1, an lncRNA transcript of MALAT1, an mRNA transcript of CGA, an miRNA transcript of MIR19B1, an miRNA transcript of MIR144, an miRNA transcript of MIR33A, an miRNA transcript of MIR130A and an mRNA of LEP;

3) at least one of whether in vitro fertilization is performed, mean arterial pressure, BMI, maternal age, parity, and abortion history.

It should be noted that the "biomarker" described in the present disclosure refers to a biochemical indicator that can be used to distinguish between preeclampsia and non-preeclampsia, including not only gene expression level but also clinical phenotype.

According to a specific embodiment of the present disclosure, the following method is used to screen and obtain molecular markers and establish a preeclampsia prediction model:

(1) Obtaining maternal plasma cell-free RNA (cfRNA)

Peripheral blood is obtained from pregnant women and immediately stored at 4°C, and plasma separation is performed within 8 hours. After plasma separation, it is immediately stored at -80°C for the next step of processing. Trizol LS is added to the plasma at a volume ratio of 1:3 and immediately shaken and mixed for extraction. The cfRNA extraction method can adopt the conventional RNA extraction method in the art, and the present disclosure does not specifically limit the cfRNA extraction method.

(2) Sequencing or RT-PCR of plasma cell-free RNA

The plasma cell-free RNA (cfRNA) library construction method is adopted. This method can simultaneously capture long and short RNA fragments in plasma, providing more features for prediction. The sequencing of cfRNA uses whole transcriptome sequencing and next-generation sequencing to sequence the plasma RNA samples of peripheral blood of pregnant women with preeclampsia and without preeclampsia. RT-PCR can also be used for analysis, including but not limited to these two methods to perform quantitative analysis of the expression profile of cfRNA.

(3) Quantification of expression profile of plasma cell-free RNA

The original cfRNA sequencing data is subjected to quality control, comprising cutting an adaptor, removing low-quality reads, and removing reads <17bp in length. The high-quality sequence after quality control is aligned to the human genome and quantified using the TPM method to obtain the expression profile of plasma cell-free RNA.

(4) Screening of risk prediction marker for preeclampsia

The expression profile of plasma cell-free RNA is used to compare the up- and down-regulated differential expression characteristics of genes in the expression profile of plasma cell-free RNA in the early and mid-pregnancy groups of pregnant women with preeclampsia and without preeclampsia, and screen out risk prediction markers for preeclampsia.

(5) Screening of molecular markers of preeclampsia based on machine learning model

Based on the molecular markers screened, a preeclampsia risk prediction model is constructed in the training set through a machine learning model (including an average neural network model (AvNN), a gradient boosting machine (GBM), a logistic regression model (LR), a neural network model (nnet) or a support vector machine (SVM)) (wherein a risk score greater than 0.5 is considered to be a high risk of preeclampsia, and less than 0.5 is considered to be a low risk of preeclampsia; and the preeclampsia risk score is automatically calculated by the model), and then the effect of the model is verified in the validation set, and the molecular markers and models with better prediction effect are screened out according to the AUC values.

(6) Construction and validation of model based on marker and clinical phenotype

According to the feature selection in step (5), a better combination of cfRNA molecular markers is obtained: an mRNA transcript of ALB, an mRNA transcript of FGA, an miRNA transcript of MIR27A, an mRNA transcript of IGFBP5, an miRNA transcript of MIR376C, an miRNA transcript of MIR215, an mRNA transcript of SERPINA1, an miRNA transcript of MIR106A, and at least one of the following:
an miRNA transcript of MIR190A, an mRNA transcript of S100A9, an mRNA transcript of APOA1, an lncRNA transcript of MALAT1, an mRNA transcript of CGA, an miRNA transcript of MIR19B1, an miRNA transcript of MIR144, an miRNA transcript of MIR33A, an miRNA transcript of MIR130A and an mRNA of LEP.
Preferably, the molecular marker combination can be an miRNA transcript of MIR190A, an mRNA transcript of ALB, an mRNA transcript of FGA, an miRNA transcript of MIR27A, an mRNA transcript of IGFBP5, an miRNA transcript of MIR376C, an mRNA transcript of S100A9, an mRNA transcript of APOA1, an lncRNA transcript of MALAT1, an miRNA transcript of MIR215, an mRNA transcript of CGA, an mRNA transcript of SERPINA1 and an miRNA transcript of MIR106A.
Preferably, the molecular marker combination can also be an miRNA transcript of MIR19B1, an mRNA transcript of ALB, an mRNA transcript of FGA, an miRNA transcript of MIR27A, an mRNA transcript of IGFBP5, an miRNA transcript of MIR376C, an miRNA transcript of MIR144, an miRNA transcript of MIR33A, an miRNA transcript of MIR130A, an miRNA transcript of MIR215, an mRNA of LEP, an mRNA transcript of SERPINA1 and an miRNA transcript of MIR106A.
Preferably, the molecular marker combination can also be an miRNA transcript of MIR130A, an miRNA transcript of MIR144, an miRNA transcript of MIR19B1, an miRNA transcript of MIR215, an miRNA transcript of MIR376C, an mRNA transcript of ALB, an mRNA transcript of FGA, an miRNA transcript of MIR27A, an mRNA of LEP, an mRNA transcript of IGFBP5, an miRNA transcript of MIR106A and an mRNA transcript of SERPINA1.
By using the combination of cfRNA molecular markers in plasma and clinical phenotype characteristics of at least one of whether in vitro fertilization is performed, mean arterial pressure, BMI, maternal age, parity, and abortion history, trained by a machine learning model, the obtained preeclampsia risk prediction model can predict the risk of preeclampsia as early as 12 weeks of pregnancy and up to 20 weeks in advance, and the prediction accuracy is high.

(7) Evaluation of effect of prediction model for preeclampsia risk based on independent samples

The model constructed in step (6) is verified by independent samples to evaluate the effect of the model.
The biomarkers obtained by screening and the preeclampsia risk prediction model obtained based on the markers disclosed in the present invention are targeted at the general asymptomatic pregnant women, regardless of whether they are high-risk or not, and can be predicted before symptoms appear. They are applicable to a wider population and have greater clinical applicability. This technology can be used in the early pregnancy (12 weeks of pregnancy) and up to 20 weeks in advance, and only requires the peripheral blood of pregnant women to be collected to predict the disease risk of preeclampsia in a non-invasive way.
The molecular markers for predicting preeclampsia obtained by screening in the present disclosure are mRNA, miRNA and lncRNA molecules in plasma, revealing the value of different types of RNA molecules in plasma in predicting preeclampsia.
Plasma cell-free RNA during pregnancy is derived from various tissues throughout the body, providing a means to monitor the health status of tissues, including reproductive tissues, such as the placenta. Based on plasma cell-free RNA, by comparing the expression level differences between the preeclampsia group and the non-preeclampsia group in the early and middle stages of pregnancy (before the disease is diagnosed), and combined with machine learning algorithms, molecular markers for predicting the risk of preeclampsia can be screened out, the risk of preeclampsia can be predicted by building a model, and the effect of the constructed preeclampsia prediction model can be evaluated by completely independent samples.

The technical solutions of the present disclosure will be explained below in conjunction with the examples. Those skilled in the art will understand that the following examples are only used to illustrate the present disclosure and should not be regarded as limiting the scope of the present disclosure. If the specific technology or conditions were not specified in the examples, they should be carried out in accordance with the technology or conditions described in the literature in the field or in accordance with the product instructions. The reagents or instruments used without indicating the manufacturer were all conventional products that could be purchased commercially.

**Examples**

**Example 1**

(1) Obtaining maternal plasma

Peripheral blood was obtained from 380 singleton pregnant women in the hospital, including 50 pregnant women with preeclampsia and 330 pregnant women without preeclampsia. The average gestational age of blood collection was 16.9 weeks (Table 1). The earliest gestational age of blood collection in the preeclampsia group was 12 weeks, and the maximum gestational age difference from blood collection to diagnosis of preeclampsia was 20 weeks. All blood samples were immediately stored at 4°C and plasma was separated within 8 hours. Plasma separation was performed by a two-step centrifugation method, centrifuging at 1,600g for 10 minutes at 4°C and then at 12,000g for 10 minutes. After plasma separation, it was immediately stored at -80°C for the next step of processing.

Table 1. Demographic and clinical phenotypes of training and validation set samples

| | Test set cases (N=35) | Test set controls (N=231) | P | Validation set cases (N=15) | Validation set controls (N=99) |
|---|---|---|---|---|---|
| Maternal age (years) | 31.8±5.94 | 28.69±4.19 | 0.000138316 | 31.8±5.15 | 29.37±3.91 |
| BMI (kg/m$^2$) | 23.61±3.54 | 21.18±3.15 | 4.21628E-05 | 25.28±3.98 | 21.75±3.29 |
| Gestational weeks at blood sampling | 15.61±2.47 | 16.87±3.87 | 0.063840609 | 18.32±4.86 | 17.55±3.68 |
| IVF rate (N, %) | 7 (20%) | 8 (3.46%) | 6.51413E-05 | 3 (20%) | 4 (4.04%) |
| Systolic blood pressure (mmHg) | 118.14±15.68 | 110.62±11.68 | 0.000950688 | 121.6±14.5 3 | 107.53±11.05 |
| Diastolic blood pressure (mmHg) | 73.91±10.77 | 66.32±10.19 | 6.90702E-05 | 77±12.96 | 66.15±8.36 |
| Mean arterial pressure | 1.09±0.14 | 0.99±0.12 | 2.90792E-05 | 1.12±0.15 | 0.98±0.098 |
| Proteinuria (N, %) | 35 (100%) | 0 (0%) | / | 15 (100%) | 0 (0%) |
| Primiparas (N, %) | 11 (31.43%) | 84 (36.36%) | 1.7454E-06 | 5 (33.33%) | 30 (30.3%) |
| Asian population (N, %) | 35 (100%) | 231 (100%) | / | 15 (100%) | 99 (100%) |
| Birth weight (g) | 1781.43 ±537.02 | 3224.94±313. 96 | 5.69319E-69 | 1760±502 | 3205.86±329.69 |
| Premature birth rate (delivery <37 weeks) (N, %) | 35 (100%) | 0 (0%) | / | 15 (100%) | 0 (0%) |

(continued)

|  | Test set cases (N=35) | Test set controls (N=231) | P | Validation set cases (N=15) | Validation set controls (N=99) |
|---|---|---|---|---|---|
| Premature onset rate (illness time <34 weeks) (N, %) | 35 (100%) | 0 (0%) | / | 15 (100%) | 0 (0%) |
| History of miscarriage (N, %) | 11 (31.42%) | 20 (8.66%) | 7.75665E-05 | 4 (26.67%) | 15 (15.15%) |
| Singleton birth rate (N, %) | 26 (74.27%) | 231 (100%) | 8.63439E-17 | 14 (93.33%) | 231 (100%) |
| Note: Mean arterial pressure = [(systolic pressure - diastolic pressure)/3 + diastolic pressure]/median | | | | | |

(2) Extraction of cfRNA

Trizol LS was added to plasma (plasma: Trizol LS volume ratio = 1:3) and immediately shaken to extract RNA.

(3) Sequencing of cfRNA

The PALM-Seq library construction method for cell-free RNA was used to sequence plasma samples from preeclampsia and non-preeclampsia cases. This method could simultaneously capture long and short RNA fragments in plasma, providing more features for prediction.

(4) Quantification of cfRNA expression profile

The original cfRNA sequencing data was subjected quality control, comprising cutting adaptor, using trimmomatic software to remove low-quality reads, and removing reads <17bp in length. The high-quality sequence after quality control was aligned to the human genome (GRCh38.p13), and the TPM method was used to quantify the expression profile of cfRNA. The formula was as follows:

$$TPM=(N_i/L_i)*1000000/(sum(N_1/L_1+ N_2/L_2+ N_3/L_3+...+ N_n/L_n))$$

$N_i$ was the number of reads aligned to the i-th gene; $L_i$ was the length of the i-th gene; $sum(N_1/L_1+N_2/L_2 + ... + N_n/L_n)$ was the sum of the values of all (n) genes after length standardization.

(5) Screening molecular markers

The expression profile of cell-free RNA was used to compare the up- and down-regulated differential expression characteristics of genes in the cfRNA expression profiles of pregnant women with preeclampsia and without preeclampsia, and to screen out markers for predicting the risk of preeclampsia.

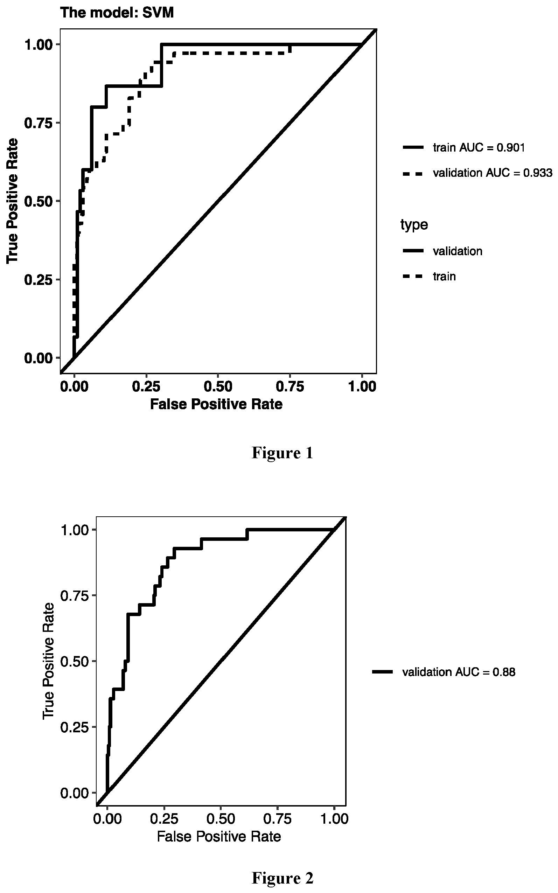

First, all groups were randomly split into training and validation sets in a ratio of 7:3. At this time, the training set contained 35 preeclampsia samples and 231 non-preeclampsia samples, and the validation set contained 15 preeclampsia samples and 99 non-preeclampsia samples (Figure 1). The screening of candidate molecular markers was completed in the training set, and the validation set was used to test the prediction effect of molecular markers and models. First, molecular markers were preliminarily screened by comparing the expression profile differences between the preeclampsia and non-preeclampsia groups. This step was implemented using the DESeq2 package (R software package). For each gene, the difference and stability of the average expression levels in the two groups were considered in this step (the absolute value of the average expression difference was greater than 1.5, the p value was less than 0.05, and the corrected p value was less than 0.1). Finally, the generalized linear model was used to screen according to the feature importance, and the molecules with higher occurrence frequency were selected as candidate molecular markers.

(6) Construction and verification of marker-based model

According to the candidate molecular markers and their combinations selected in step (5), the model was constructed. First, in the training set, based on the selected candidate molecular markers, the support vector machine (SVM) as machine learning algorithm was used to assess the risk of preeclampsia. The algorithm used a ten-fold cross-validation method to select the optimal parameters for the construction of prediction model. By comparing the results of the model training set and the validation set, the RNA molecular combinations with AUC (Area under the receiver operating characteristic curve) of the training set and the validation set < 0.9 were removed to obtain the optimized RNA molecular marker combination. Then, the model effect was verified in the validation set, and the models were sorted according to the AUC values to screen out the model with better prediction effect and the corresponding molecular marker combination.

(7) Construction of model based on markers and clinical phenotypes

According to step (6), the model with the largest AUC value was selected to obtain the combination of best 13 cfRNA molecular markers (Table 5). Using these 13 cfRNA molecular markers in combination with two clinical phenotypes, whether in vitro fertilization is performed and mean arterial pressure, a preeclampsia prediction model was constructed using the SVM model in 380 samples (50 preeclampsia pregnant women and 330 non-preeclampsia pregnant women) (the modeling method was the same as step (6)). For the obtained model, the training set AUC was 0.901, the validation set AUC was 0.933 (Figure 1), and the specificity was 99% (Table 2).

(8) Evaluation of effect of molecular markers on prediction model for preeclampsia risk

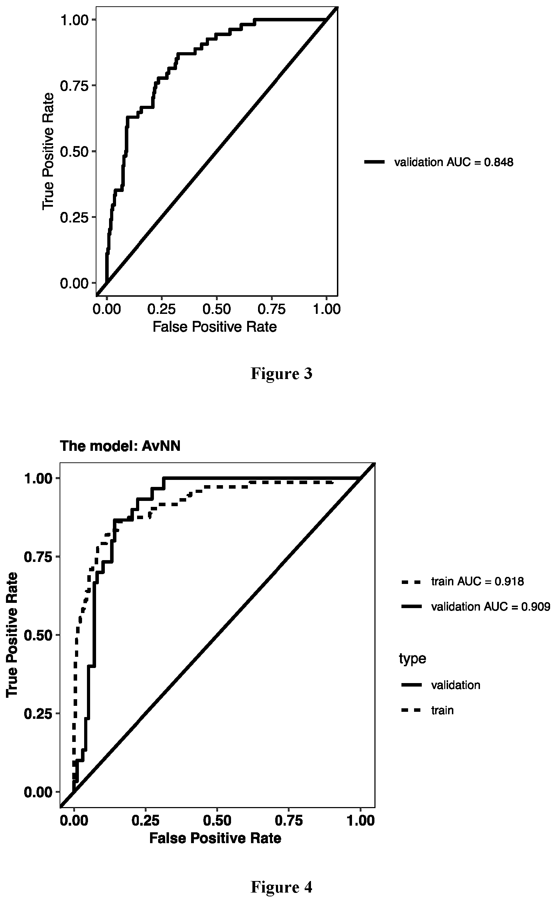

The SVM preeclampsia prediction model constructed in step (7) was further independently verified using two completely independent groups of samples to evaluate the model effect.
The model was validated in the first group of 262 independent samples (28 pregnant women with preeclampsia and 234 pregnant women without preeclampsia), and the AUC of the independent validation set was 0.88 (Figure 2), with a specificity of 98.7%, a negative predictive value (NPV) of 92.04%, and a positive predictive value (PPV) of 72.73% (Table 3), which was higher than the existing technology level (PPV=32%, RNA profiles reveal signatures of future health and disease in pregnancy. Nature (2022)).
The model was validated in the second group of 288 independent samples (54 pregnant women with preeclampsia and 234 pregnant women without preeclampsia), and the AUC of the independent validation set was 0.848 (Figure 3), with a specificity of 99.14%, a negative predictive value (NPV) of 83.76%, and a positive predictive value (PPV) of 81.82% (Table 4), which was higher than the existing technology level (PPV=32%).

Table 2. AUC and specificity of SVM model in test set and validation set

|  | Diseases | Controls | Test set AUC | Validation set AUC | Validation set specificity (%) |
|---|---|---|---|---|---|
| Test set | 35 | 231 | 0.901 | - | - |
| Validation set | 15 | 99 | - | 0.933 | 99% |

Table 3. Evaluation of effect of prediction model for preeclampsia risk (independent validation in the first group of 262 cases)

|  | Diseases | Control s | Validation set AUC | Specificity (%) | Negative predictive value (%) | Positive predictive value (%) |
|---|---|---|---|---|---|---|
| Validation set | 28 | 234 | 0.88 | 98.7% | 92.04% | 72.73% |

Table 4. Evaluation of effect of prediction model for preeclampsia risk (independent validation in the second group of 288 cases)

|  | Diseases | Controls | Validation set AUC | Specificity (%) | Negative predictive value (%) | Positive predictive value (%) |
|---|---|---|---|---|---|---|
| Validation set | 54 | 234 | 0.848 | 99.14% | 83.76% | 81.82% |

Table 5. Gene and transcript information of 13 preeclampsia characteristic genes

| Gene name | Gene ID | Gene sequence length (bp) | RNA type | Sequence No. |
|---|---|---|---|---|
| MIR190A | 406965 | 85 | miRNA | SEQ ID NO:9 |
| ALB | 213 | 17196 | mRNA | SEQ ID NO:1 |

(continued)

| Gene name | Gene ID | Gene sequence length (bp) | RNA type | Sequence No. |
|---|---|---|---|---|
| FGA | 2243 | 7617 | mRNA | SEQ ID NO:2 |
| MIR27A | 407018 | 78 | miRNA | SEQ ID NO:3 |
| IGFBP5 | 3488 | 23445 | mRNA | SEQ ID NO:4 |
| MIR376C | 442913 | 66 | miRNA | SEQ ID NO:5 |
| S100A9 | 6280 | 3170 | mRNA | SEQ ID NO:10 |
| APOA1 | 335 | 2200 | mRNA | SEQ ID NO:11 |
| MALAT1 | 378938 | 8779 | lncRNA | SEQ ID NO:12 |
| MIR215 | 406997 | 110 | miRNA | SEQ ID NO:6 |
| CGA | 1081 | 9606 | mRNA | SEQ ID NO:13 |
| SERPINA1 | 5265 | 13889 | mRNA | SEQ ID NO:7 |
| MIR106A | 406899 | 81 | miRNA | SEQ ID NO:8 |

The gene ID numbers in the table were the gene ID numbers displayed in the NCBI database.

**Example 2**

(1) Obtaining maternal plasma

Peripheral blood samples from 430 singleton pregnant women were obtained from the hospital, including 100 pregnant women with preeclampsia and 330 pregnant women without preeclampsia. The average gestational age of blood collection was 16.9 weeks (Table 6). The earliest gestational age of blood collection in the preeclampsia group was 12 weeks, and the maximum gestational age difference from blood collection to diagnosis of preeclampsia was 20 weeks. All blood samples were immediately stored at 4°C and subjected to plasma separation within 8 hours. A two-step centrifugation method was used for plasma separation, centrifuging at 1,600g for 10 minutes at 4°C and then at 12,000g for 10 minutes. After plasma separation, it was immediately stored at -80°C for further processing.

Table 6. Demographic and clinical phenotypes of samples in training and validation sets

| | Test set cases (N=70) | Test set controls (N=231) | P | Validation set cases (N=30) | Validation set controls (N=99) |
|---|---|---|---|---|---|
| Maternal age (years) | 31.82±5.54 | 28.94±3.98 | 2.14926E-06 | 30±4.18 | 28.80±4.30 |
| BMI (kg/m$^2$) | 24.19±3.59 | 21.42±2.88 | 1.59063E-10 | 23.37±3.51 | 21.42±3.21 |
| Gestational weeks at blood sampling | 16.49±3.82 | 16.98±3.85 | 0.356519348 | 16.54±4.38 | 17.30±3.76 |
| IVF rate (N, %) | 11 (15.71%) | 8 (3.46%) | 0.000167693 | 4 (13.33%) | 4 (4.04%) |
| Systolic blood pressure (mmHg) | 118.01±14.21 | 109.62±11.7 8 | 9.93896E-07 | 115.2±14.11 | 108.31±15.06 |
| Diastolic blood pressure (mmHg) | 73.82±10.98 | 66.67±9.79 | 3.67229E-07 | 70.6±9.46 | 64.46±11.05 |
| Mean arterial pressure | 1.09±0.14 | 0.99±0.11 | 2.16463E-08 | 1.05±0.12 | 0.97±0.14 |
| Proteinuria (N, %) | 70 (100%) | 0 (0%) | / | 70 (100%) | 0 (0%) |

(continued)

|  | Test set cases (N=70) | Test set controls (N=231) | P | Validation set cases (N=30) | Validation set controls (N=99) |
|---|---|---|---|---|---|
| Primiparas (N, %) | 21 (30%) | 73 (31.6%) | 0.116955094 | 5 (16.67%) | 41 (41.41%) |
| Asian population (N, %) | 70 (100%) | 231 (100%) | / | 30 (100%) | 99 (100%) |
| Birth weight (g) | 2142.76 ±598.33 | 3217.14±32 6.9 | 2.65929E-59 | 1959.17±553.0 6 | 3224.04±299.2 8 |
| Premature birth rate (delivery <37 weeks) (N, %) | 70 (100%) | 0 (0%) | / | 30 (100%) | 0 (0%) |
| History of miscarriage (N, %) | 17 (24.29%) | 28 (12.12%) | 0.016605451 | 5 (16.67%) | 7 (7.07%) |
| Singleton birth rate (N, %) | 53 (75.71%) | 231 (100%) | 1.53569E-15 | 24 (80%) | 231 (100%) |
| Note: Mean arterial pressure = [(systolic pressure - diastolic pressure) / 3 + diastolic pressure] / median | | | | | |

(2) Extraction of cfRNA

Trizol LS was added to plasma (plasma: Trizol LS volume ratio = 1:3) and immediately shaken to mix and extract cfRNA.

(3) Sequencing of cfRNA

The cell-free RNA library construction method PALM-Seq was used to sequence plasma samples of preeclampsia and non-preeclampsia. This method could simultaneously capture long and short RNA fragments in plasma, providing more features for prediction.

(4) Quantification of cfRNA expression profile

The original cfRNA sequencing data was subjected to quality control, comprising cutting adaptor, using trimmomatic software to remove low-quality reads, and removing reads <17bp in length. The high-quality sequence after quality control was aligned to the human genome (GRCh38.p13), and the TPM method was used to quantify the expression profile of cfRNA. The formula was as follows:

$$TPM=(N_i/L_i)*1000000/(sum(N_1/L_1+ N_2/L_2+ N_3/L_3+\ldots+ N_n/L_n))$$

$N_i$ was the number of reads aligned to the i-th gene; $L_i$ was the length of the i-th gene; $sum(N_1/L_1+N_2/L_2 + ... + N_n/L_n)$ was the sum of the values of all (n) genes after length standardization.

(5) Screening molecular markers

The expression profile of cell-free RNA was used to compare the up- and down-regulated differential expression characteristics of genes in the cfRNA expression profiles of pregnant women with preeclampsia and without preeclampsia, and to screen out markers for predicting the risk of preeclampsia.

First, all groups were randomly split into training and validation sets in a ratio of 7:3. At this time, the training set contained 70 preeclampsia samples and 231 non-preeclampsia samples, and the validation set contained 30 preeclampsia samples and 99 non-preeclampsia samples (Figure 4). All molecular marker screening was completed in the training set, and the validation set was used to test the prediction effect of molecular markers and models. First, the candidate molecular markers were preliminarily screened by comparing the expression profile differences between the preeclampsia and non-preeclampsia groups. This step was implemented using the DESeq2 package (R software package). For each gene, the difference and stability of the average expression levels in the two groups were considered in this step (the absolute value of the average expression level difference was greater than 1.5, the p value was less than 0.05, and the corrected p value was less than 0.1). Finally, the generalized linear model was sued for screening according to the feature importance, and the molecules with higher occurrence frequency were selected as candidate molecular markers.

(6) Construction and verification of marker-based model

Based on the selected molecular markers, the model was constructed. First, in the training set, based on the selected molecular markers, the average neural network model (AvNN) as machine learning algorithm was used to assess the risk of preeclampsia. The algorithm used a ten-fold cross-validation method to select the optimal parameters for the construction of prediction model (the expression levels of molecular markers were used as input, and the risk scores of pregnant women with preeclampsia were used as output to train the model, wherein a risk score greater than or equal to 0.5 was considered to be a high risk of preeclampsia, and a risk score less than 0.5 was considered to be a low risk of preeclampsia). The results of the model training set and the validation set were compared, and the RNA molecular combinations with AUC (area under the receiver operating characteristic curve) of less than 0.9 in the training set and validation set were removed to obtain the optimized RNA molecular marker combination. The model effect was then verified in the validation set, and by the models were sorted according to the AUC values to screen out the model with better prediction effect and the corresponding molecular marker combination.

(7) Construction and validation of model based on markers and clinical phenotypes

According to step (6), the model with the largest AUC value was selected to obtain the combination of best 13 cfRNA molecular markers (Table 10). Using these 13 cfRNA molecular markers in combination with two clinical phenotypes, whether in vitro fertilization is performed and mean arterial pressure, the AvNN model was used to construct a preeclampsia prediction model in 430 samples (100 pregnant women with preeclampsia and 330 pregnant women without preeclampsia) (the modeling method was the same as step (6)), in which the model training set AUC = 91.8%, the validation set AUC = 90.9% (Figure 4), and the specificity was 92.9% (Table 7).

(8) Evaluation of effect of prediction model for preeclampsia risk based on independent samples

The AvNN preeclampsia prediction model constructed in step (7) was further independently validated using two completely independent groups of samples to evaluate the model effect.

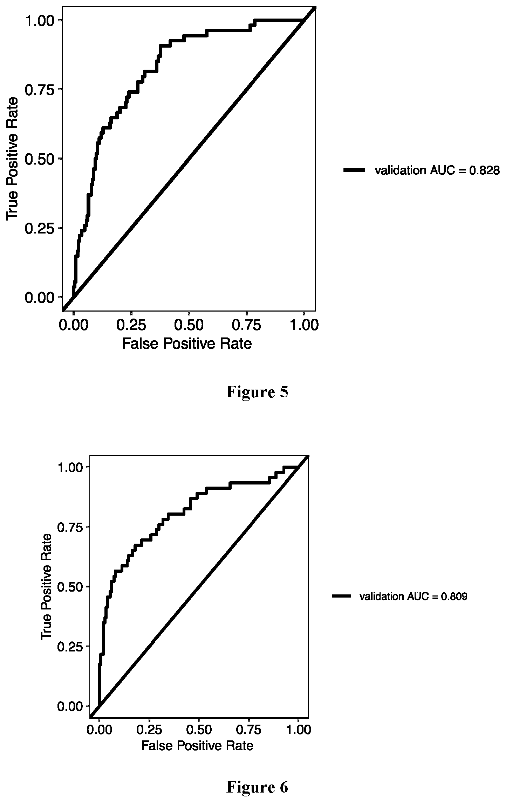

First, the model was validated in the first group of 288 independent samples (54 pregnant women with preeclampsia and 234 pregnant women without preeclampsia), and the AUC of the independent validation set was 0.828 (Figure 5), with a specificity of 88%, a negative predictive value (NPV) of 90.4%, and a positive predictive value (PPV) of 53.3% (Table 8), which was higher than the existing technical level (PPV=32%).

Then, the model was validated in the second group of 197 independent samples (46 pregnant women with preeclampsia and 151 pregnant women without preeclampsia), and the AUC of the independent validation set was 0.809 (Figure 6), with a specificity of 92.5%, a negative predictive value (NPV) of 87.4%, and a positive predictive value (PPV) of 68.4% (Table 9), which was higher than the existing technical level (PPV=32%).

Table 7. AUC and specificity of AvNN model in test set and validation set

|  | Diseases | Controls | Test set AUC | Validation set AUC | Validation set specificity (%) |
|---|---|---|---|---|---|
| Test set | 70 | 231 | 0.918 | - | - |
| Validation set | 30 | 99 | - | 0.909 | 92.9% |

Table 8. Evaluation of effect of prediction model for preeclampsia risk (independent validation in the first group of 288 cases)

|  | Diseases | Controls | Validation set AUC | Specificity (%) | Negative predictive value (%) | Positive predictive value (%) |
|---|---|---|---|---|---|---|
| Validation set | 54 | 234 | 0.828 | 88% | 90.4% | 53.3% |

Table 9. Evaluation of effect of prediction model for preeclampsia risk (independent validation in the second group of 197 cases)

| | Disease | Control | Validation set AUC | Specificity (%) | Negative predictive value (%) | Positive predictive value (%) |
|---|---|---|---|---|---|---|
| Validation set | 46 | 151 | 0.809 | 92.5% | 87.4.4% | 68.4% |

Table 10. Gene and transcript information of 13 preeclampsia characteristic genes

| Gene name | Gene ID | Gene sequence length (bp) | Transcript type | Sequence No. |
|---|---|---|---|---|
| MIR19B1 | 406980 | 87 | miRNA | SEQ ID NO:17 |
| ALB | 213 | 17196 | mRNA | SEQ ID NO:1 |
| FGA | 2243 | 7617 | mRNA | SEQ ID NO:2 |
| MIR27A | 407018 | 78 | miRNA | SEQ ID NO:3 |
| IGFBP5 | 3488 | 23445 | mRNA | SEQ ID NO:4 |
| MIR376C | 442913 | 66 | miRNA | SEQ ID NO:5 |
| MIR144 | 406936 | 86 | miRNA | SEQ ID NO:16 |
| MIR33A | 407039 | 69 | miRNA | SEQ ID NO:18 |
| MIR130A | 406919 | 89 | miRNA | SEQ ID NO:15 |
| MIR215 | 406997 | 110 | miRNA | SEQ ID NO:6 |
| LEP | 3952 | 16352 | mRNA | SEQ ID NO:14 |
| SERPINA1 | 5265 | 13889 | mRNA | SEQ ID NO:7 |
| MIR106A | 406899 | 81 | miRNA | SEQ ID NO:8 |

**Example 3**

In this example, the effect of biomarkers on prediction model for preeclampsia risk was evaluated according to the method in Example 2, except that the molecular markers and clinical phenotypes were different. Specifically, the clinical phenotypes of this example were the following 6 clinical phenotypes: whether in vitro fertilization is performed, mean arterial pressure, BMI, maternal age, parity, and history of miscarriage, and the 12 molecular markers in this example were: MIR130A, MIR144, MIR19B1, MIR215, MIR376C, ALB, FGA, MIR27A, LEP, IGFBP5, MIR106A and SERPINA1. The AvNN model was used to construct model for the 12 molecular markers and 6 clinical phenotypes.

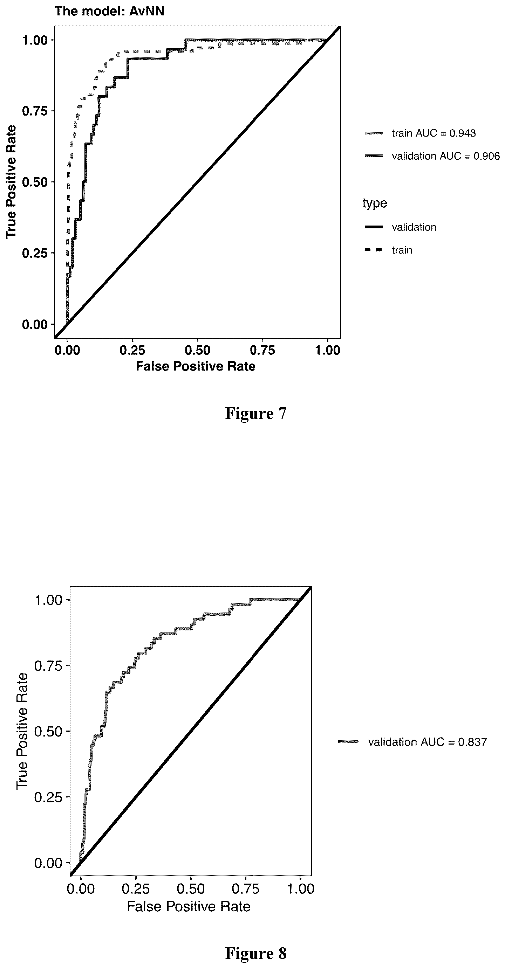

The model training set AUC = 94.3%, the validation set AUC = 90.6% (Figure 7), and the specificity was 90% (Table 11).

The AvNN preeclampsia prediction model constructed above was further independently validated using a group of completely independent samples to evaluate the model effect. The model was validated in the first group of 288 independent samples (54 pregnant women with preeclampsia and 234 pregnant women without preeclampsia).

The AUC of the independent validation set could reach 0.837 (Figure 8), with a specificity of 89.7%, a negative predictive value (NPV) of 90%, and a positive predictive value (PPV) of 53.9% (Table 12), which was higher than the existing technical level.

Table 11. AUC and specificity of AvNN model in test set and validation set

| | Diseases | Controls | Test set AUC | Validation set AUC | Validation set specificity (%) |
|---|---|---|---|---|---|
| Test set | 70 | 231 | 0.943 | - | - |
| Validation set | 30 | 99 | - | 0.906 | 90% |

Table 12. Evaluation of effect of prediction model for preeclampsia risk (independent validation in the first group of 288 cases)

|  | Diseases | Controls | Validation set AUC | Specificity (%) | Negative predictive value (%) | Positive predictive value (%) |
|---|---|---|---|---|---|---|
| Validation set | 54 | 234 | 0.837 | 89.7% | 90% | 53.9% |

**Comparative Example 1**

In this example, the candidate molecular markers obtained by selection according to step (5) of Example 1 were combined, and the GBM model was used to construct the model according to the method of step (6). The molecular marker combination was MIR130A, MIR144, MIR19B1, MIR215 and MIR376C.

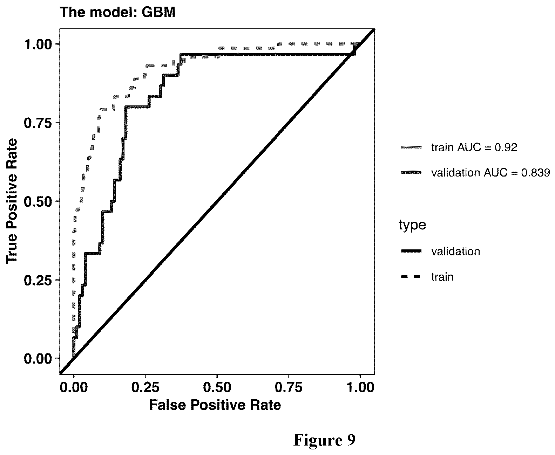

The model training set AUC = 92%, the validation set AUC = 83.9% (Figure 9), and the specificity was 96% (Table 13).

Table 13. Evaluation of effect of prediction model for preeclampsia risk

|  | Diseases | Controls | Test set AUC | Validation set AUC | Validation set specificity (%) |
|---|---|---|---|---|---|
| Test set | 35 | 231 | 0.92 | - | - |
| Validation set | 15 | 99 | - | 0.839 | 96% |

In the description of the description, the reference terms "one example", "some examples", "embodiment", "specific example" or "some embodiments" refer to the specific features, structures, materials or characteristics described in conjunction with the example or embodiment included in at least one example or embodiment of the present disclosure. In the description, the schematic representation of the above terms does not necessarily refer to the same embodiment or example. Moreover, the specific features, structures, materials or characteristics described can be combined in any one or more embodiments or examples in a suitable manner. In addition, those skilled in the art can combine different embodiments or examples described in the description and the features of different embodiments or examples without contradiction.

Although the examples or embodiments of the present disclosure have been shown and described above, it is understood that the above examples or embodiments are exemplary and cannot be construed as limiting the present disclosure. A person skilled in the art may make changes, modifications, substitutions and modifications to the above examples or embodiments within the scope of the present disclosure.

**Claims**

1. A biomarker combination for detecting preeclampsia or predicting the risk of preeclampsia, comprising the following genes:
ALB, FGA, MIR27A, IGFBP5, MIR376C, MIR215, SERPINA1 and MIR106A.

2. The biomarker combination according to claim 1, wherein the biomarker combination is a combination of RNA molecular markers;
preferably, the biomarker combination is a combination of cell-free RNA molecular markers.

3. The biomarker combination according to claim 1 or 2, wherein the biomarker combination comprises:
an mRNA transcript of ALB, an mRNA transcript of FGA, an miRNA transcript of MIR27A, an mRNA transcript of IGFBP5, an miRNA transcript of MIR376C, an miRNA transcript of MIR215, an mRNA transcript of SERPINA1 and an miRNA transcript of MIR106A.

4. The biomarker combination according to any one of claims 1 to 3, wherein the biomarker combination further comprises at least one of the following genes:

MIR190A, S100A9, APOA1, MALAT1, CGA, LEP, MIR130A, MIR144, MIR19B1 and MIR33A;
preferably, the biomarker combination further comprises at least one of the following:
an miRNA transcript of MIR190A, an mRNA transcript of S100A9, an mRNA transcript of APOA1, an lncRNA transcript of MALAT1, an mRNA transcript of CGA, an miRNA transcript of MIR19B1, an miRNA transcript of MIR144, an miRNA transcript of MIR33A, an miRNA transcript of MIR130A and an mRNA of LEP.

5. The biomarker combination according to any one of claims 1 to 4, wherein the biomarker combination comprises:

MIR190A, ALB, FGA, MIR27A, IGFBP5, MIR376C, S100A9, APOA1, MALAT1, MIR215, CGA, SERPINA1 and MIR106A;
preferably, the biomarker combination comprises: an miRNA transcript of MIR190A, an mRNA transcript of ALB, an mRNA transcript of FGA, an miRNA transcript of MIR27A, an mRNA transcript of IGFBP5, an miRNA transcript of MIR376C, an mRNA transcript of S100A9, an mRNA transcript of APOA1, an lncRNA transcript of MALAT1, an miRNA transcript of MIR215, an mRNA transcript of CGA, an mRNA transcript of SERPINA1 and an miRNA transcript of MIR106A.

6. The biomarker combination according to any one of claims 1 to 4, wherein the biomarker combination comprises:

MIR19B1, ALB, FGA, MIR27A, IGFBP5, MIR376C, MIR144, MIR33A, MIR130A, MIR215, LEP, SERPINA1 and MIR106A;
preferably, the biomarker combination comprises: an miRNA transcript of MIR19B1, an mRNA transcript of ALB, an mRNA transcript of FGA, an miRNA transcript of MIR27A, an mRNA transcript of IGFBP5, an miRNA transcript of MIR376C, an miRNA transcript of MIR144, an miRNA transcript of MIR33A, an miRNA transcript of MIR130A, an miRNA transcript of MIR215, an mRNA of LEP, an mRNA transcript of SERPINA1 and an miRNA transcript of MIR106A.

7. The biomarker combination according to any one of claims 1 to 6, wherein the biomarker combination further comprises: a clinical phenotype;
preferably, the clinical phenotype comprises at least one of the followings:
whether in vitro fertilization is performed, mean arterial pressure, BMI, maternal age, parity and abortion history.

8. A reagent for detecting the biomarker combination according to any one of claims 1 to 6, wherein the reagent comprises: a biomolecule capable of specifically hybridizing with any one of the biomarkers in the biomarker combination or their expression products and/or a detection reagent that uses the biomarker combination as a detection target.

9. The reagent according to claim 8, wherein the biomolecule comprises at least one of a primer, a probe, and an antibody.

10. A detection product, comprising the reagent according to claim 8 or 9.

11. The detection product according to claim 10, wherein the detection product comprises a kit and/or a chip.

12. A method for detecting preeclampsia or predicting the risk of preeclampsia, wherein the method comprises:

(1) obtaining a biological sample to be detected;
(2) determining the detection data of the biomarker combination according to any one of claims 1 to 7 in the biological sample obtained in step (1);
(3) identifying whether there is a preeclampsia or a risk of preeclampsia based on the detection data as described in step (2),

wherein the detection data comprises the expression level of a gene and optionally the result of clinical phenotype.

13. The method according to claim 12, wherein the biological sample comprises one or more of plasma, whole blood, amniotic fluid, serum and urine;

preferably, the biological sample is collected before the 33$^{rd}$ gestational week of a pregnant woman;
preferably, the biological sample is derived from a peripheral blood sample of the pregnant woman.

14. The method according to claim 12 or 13, wherein the identification of whether there is a preeclampsia or a risk of preeclampsia is achieved by a prediction model;

preferably, the prediction model uses the detection data of the biomarker combination of preeclampsia samples and non-preeclampsia samples as input, and uses the risk score of preeclampsia risk as output, and is obtained by machine learning model training;

preferably, the machine learning model comprises: one or more of an average neural network model, a gradient boosting machine, a logistic regression model, a neural network model, and a support vector machine;

preferably, the expression level of genes is the RNA expression level of genes.

15. A device for detecting preeclampsia or predicting the risk of preeclampsia, comprising:

an acquisition module for acquiring a biological sample to be detected;

a detection module for determining the detection data of the biomarker combination according to any one of claims 1 to 7 in the biological sample;

an identification module for identifying whether there is a preeclampsia or a risk of preeclampsia based on the data of the biomarker combination;

wherein, the detection data of the biomarker combination comprises the expression level of a gene and optionally the result of clinical phenotype.

16. A device for detecting preeclampsia or predicting the risk of preeclampsia, comprising a memory for storing a program; a processor for executing the program stored in the memory to implement the method according to any one of claims 12 to 14.

17. A computer-readable storage medium, on which a program is stored, and the program can be executed by a processor to implement the method according to any one of claims 12 to 14.

18. Use of a biomarker as a target for screening a drug for treating or preventing preeclampsia, wherein the biomarker comprises the biomarker combination according to any one of claims 1 to 6.

19. Use of a biomarker in detecting preeclampsia or predicting the risk of preeclampsia, wherein the biomarker comprises the biomarker combination according to any one of claims 1 to 7.

**The model: SVM**

train AUC = 0.901

validation AUC = 0.933

type

validation

train

**Figure 1**

validation AUC = 0.88

**Figure 2**

**Figure 3**

**Figure 4**

**Figure 5**

**Figure 6**

Figure 7

Figure 8

The model: GBM

Figure 9

## INTERNATIONAL SEARCH REPORT

| International application No. |
| --- |
| **PCT/CN2022/139798** |

**A. CLASSIFICATION OF SUBJECT MATTER**

C12Q1/68(2018.01)i

According to International Patent Classification (IPC) or to both national classification and IPC

**B. FIELDS SEARCHED**

Minimum documentation searched (classification system followed by classification symbols)

IPC: C12Q

Documentation searched other than minimum documentation to the extent that such documents are included in the fields searched

Electronic data base consulted during the international search (name of data base and, where practicable, search terms used)

CNTXT, VEN, ENTXT, CNKI, 万方数据库, WANFANG Database, ISI, NCBI: 子痫, 先兆子痫, eclampsia, preeclampsia, 标志物, biomarker, ALB, FGA, MIR27A, MIR376C, MIR215, SERPINA1, MIR106A, MIR190A, S100A9, APOA1, MALAT1, CGA, LEP, MIR130A, MIR144, MIR19B1, MIR33A, 发明人

**C. DOCUMENTS CONSIDERED TO BE RELEVANT**

| Category* | Citation of document, with indication, where appropriate, of the relevant passages | Relevant to claim No. |
| --- | --- | --- |
| X | US 2018356405 A1 (ESSENLIX CORP.) 13 December 2018 (2018-12-13) claims 1, 12, 18 and 24, and description, paragraphs 161 and 165, and tables 1-3 and 7 | 1-11 |
| Y | US 2018356405 A1 (ESSENLIX CORP.) 13 December 2018 (2018-12-13) claims 1, 12, 18 and 24, and description, paragraphs 161 and 165, and tables 1-3 and 7 | 4-11 |
| A | CN 113677807 A (ILLUMINA, INC.) 19 November 2021 (2021-11-19) claim 1, and description, paragraphs 167-170 | 1-19 |
| A | CN 104487593 A (THE BOARD OF TRUSTEES OF THE LELAND STANFORD JUNIOR UNIVERSITY) 01 April 2015 (2015-04-01) claims | 1-19 |
| A | CN 113567687 A (TIANJIN YUNJIAN MEDICAL DEVICE CO., LTD. et al.) 29 October 2021 (2021-10-29) claims, and description, paragraph 54 | 1-19 |
| A | CN 114941025 A (SECOND AFFILIATED HOSPITAL & YUYING CHILDREN'S HOSPITAL OF WENZHOU MEDICAL UNIVERSITY) 26 August 2022 (2022-08-26) claims | 1-19 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 September 2023** | **07 September 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2022/139798**

### C. DOCUMENTS CONSIDERED TO BE RELEVANT

| Category* | Citation of document, with indication, where appropriate, of the relevant passages | Relevant to claim No. |
|---|---|---|
| T | ZHOU, Si et al. "Noninvasive preeclampsia prediction using plasma cell-free RNA signatures" *AMERICAN JOURNAL OF OBSTETRICS AND GYNECOLOGY,* 19 May 2023 (2023-05-19), pages 1.e1-1.e16 | 1-19 |
| Y | WANG, Ying et al. "Potential of immune-related genes as biomarkers for diagnosis and subtype classification of preeclampsia" *FRONTIERS IN GENETICS,* Vol. 11, 01 December 2020 (2020-12-01), document 579709, pages 1-12 | 4-11 |
| A | 包丹 等 (BAO, Dan et al.). "子痫前期风险预测指标的研究进展 (Non-official translation: Research Progress on Risk Predictors of Preeclampsia)" 中国临床研究 *(Chinese Journal of Clinical Research),* Vol. 33, No. 10, 31 October 2020 (2020-10-31), pages 1435-1438 | 1-19 |

Form PCT/ISA/210 (second sheet) (July 2022)

**INTERNATIONAL SEARCH REPORT**

International application No.

**PCT/CN2022/139798**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2022/139798**

| Patent document cited in search report | | | Publication date (day/month/year) | Patent family member(s) | | | Publication date (day/month/year) |
|---|---|---|---|---|---|---|---|
| US | 2018356405 | A1 | 13 December 2018 | WO | 2017058827 | A1 | 06 April 2017 |
| | | | | US | 2023013771 | A1 | 19 January 2023 |
| CN | 113677807 | A | 19 November 2021 | CA | 3131748 | A1 | 27 May 2021 |
| | | | | EP | 4010495 | A1 | 15 June 2022 |
| | | | | AU | 2020387423 | A1 | 30 September 2021 |
| | | | | US | 2023135486 | A1 | 04 May 2023 |
| | | | | US | 2021155987 | A1 | 27 May 2021 |
| | | | | JP | 2023501760 | A | 19 January 2023 |
| | | | | WO | 2021102236 | A1 | 27 May 2021 |
| CN | 104487593 | A | 01 April 2015 | HK | 1206071 | A1 | 31 December 2015 |
| | | | | GB | 201420279 | D0 | 31 December 2014 |
| | | | | GB | 2515983 | A | 07 January 2015 |
| | | | | HK | 1206790 | A1 | 15 January 2016 |
| | | | | WO | 2013169751 | A1 | 14 November 2013 |
| | | | | EP | 2847354 | A1 | 18 March 2015 |
| | | | | EP | 2847354 | A4 | 30 December 2015 |
| | | | | US | 2015099655 | A1 | 09 April 2015 |
| | | | | JP | 2015519564 | A | 09 July 2015 |
| CN | 113567687 | A | 29 October 2021 | None | | | |
| CN | 114941025 | A | 26 August 2022 | None | | | |

Form PCT/ISA/210 (patent family annex) (July 2022)

## REFERENCES CITED IN THE DESCRIPTION

**Patent documents cited in the description**

- CN 110785499 A

- CN 104412107 B

**Non-patent literature cited in the description**

- **POON LC** ; **SHENNAN A** ; **HYETT JA et al.** The International Federation of Gynecology and Obstetrics (FIGO) initiative on preeclampsia: A pragmatic guide for first-trimester screening and prevention.. *Int J Gynaecol Obstet.*, 2019, vol. 145 (1), 1-33
- Obstetricians ACo and Gynecologists. Gestational hypertension and preeclampsia: ACOG Practice Bulletin. *Obstet Gynecol.*, 2020, vol. 135 (222), e237-e60

- **VILLA PM** ; **KAJANTIE E** ; **RÄIKKÖNEN K et al.** Aspirin in the prevention of preeclampsia in high-risk women: a randomised placebo-controlled PREDO Trial and a meta-analysis of randomised trials.. *BJOG: An International Journal of Obstetrics & Gynaecology.*, 2013, vol. 120, 64-74